Europäisches Patentamt

**European Patent Office**

Office européen des brevets

(11) Numéro de publication: **0 137 007 B1**

# (12) FASCICULE DE BREVET EUROPEEN

(45) Date de publication du fascicule du brevet:
08.11.89

(51) Int. Cl.⁴: **A 61 N 1/36**

(21) Numéro de dépôt: **84900731.5**

(22) Date de dépôt: **17.02.84**

(86) Numéro de dépôt international:
**PCT/FR 84/00033**

(87) Numéro de publication internationale:
**WO 84/03219 (30.08.84 Gazette 84/21)**

(54) **DISPOSITIF ELECTRONIQUE A PROGRAMMATION, DESTINE A STIMULER ET A CONTROLER LA CONTRACTION DES MUSCLES, NOTAMMENT DES MUSCLES LISSES DU TISSU VASCULAIRE.**

(30) Priorité: **18.02.83 FR 8302631**

(43) Date de publication de la demande:
**17.04.85 Bulletin 85/16**

(45) Mention de la délivrance du brevet:
**08.11.89 Bulletin 89/45**

(84) Etats contractants désignés:
**AT BE CH DE FR GB LI LU NL SE**

(56) Documents cités:
**DE-A- 2 903 392**
**DE-A- 3 049 530**
**FR-A- 2 418 646**
**FR-A- 2 433 950**
**US-A- 4 255 790**

**IEEE 1981 IECI Proceedings (Conference report: "Applications of Mini and Microcomputers" held in San Francisco, on 8-12 November 1981) (New York, US) A. Cohen et al.: "A microcomputer-based, multi-channel, fonctional neuromuscular stimulator unit", pp. 76-81**

(73) Titulaire: **TAGE S.A., Allée de la Victoire Aigremont, F-78240 Chambourcy (FR)**

(72) Inventeur: **Klotz, Antoine, 1, Résidence Coeur de Maule, F-78580 Maule (FR)**

(74) Mandataire: **Tanguy, Gilbert André, 8, rue Cépré, F-75015 Paris (FR)**

ACTORUM AG

## Description

La présente invention a pour objet un dispositif électronique à programmation, destiné à stimuler et à contrôler la contraction des muscles, notamment des muscles lisses du tissu vasculaire.

Le dispositif a une action thérapeutique certaine dans le traitement de la maladie thromboembolique et de la maladie phlébitique dont l'étendue est considérable dans le monde entier. A titre indicatif de leur importance, en 1981 aux U.S.A. sur plus de 600 000 personnes atteintes de maladie thrombotique, 214 440 sont mortes d'embolie pulmonaire. En France, comme en Angleterre, ce nombre se situait entre 40 et 50 000 morts, au Japon environ 100 000 morts.

Les modes de prévention sont essentiellement chimiothérapiques avec l'usage d'anticoagulants, d'antiagrégants plaquettaires et autres produits associés. Pour en surveiller l'application, il convient de recourir à de nombreux et coûteux tests biologiques. Enfin, comme les chiffres cités précédemment l'indiquent, leur efficacité est loin d'être probante. Leur seul coût direct en France pour les hôpitaux publics dépasse 2 milliards de Francs par an.

Pour la maladie phlébitique, un affaiblissement du muscle vasculaire peut provoquer la déchirure irréparable des cellules et la formation de varices. Des formes moins aiguës de cette maladie sont constatées dans les syndromes orthostatiques, pathologie des jambes lourdes, etc... Le médecin se trouve relativement désarmé devant ces maladies, les tests cliniques démontrant la relative inefficacité de la chimiothérapie. Cependant 887 millions de Francs ont été dépensés en 1981 pour l'achat de phlébotoniques en France.

Les recherches médicales et leur publication, menées aux U.S.A., en Australie, au Royaume-Uni, en France, toutes tendent à stimuler la circulation de retour pour lutter contre ces phénomènes. Ainsi ont été réalisées des bottes pneumatiques compressant et décompressant les membres inférieurs de façon rythmée pour obtenir ce flux. Cela permet des résultats intéressants.

Des appareils électriques ont parfois été réalisés pour stimuler le triceps sural (Pollock) mais ne pouvaient être utilisés le malade étant réveillé.

Dans l'art antérieur, on a également déjà décrit un certain nombre d'appareils destinés à stimuler les muscles striés. Parmi ces derniers, on peut citer l'appareil décrit dans le brevet allemand n° 976 354 (GRATZEL) qui se rapporte à la stimulation des muscles striés au moyen d'impulsions électriques. De même, le brevet français n° 2 296 437 (BAULANDE) concerne un appareil et un procédé pour stimuler la contraction des muscles striés par l'utilisation d'impulsions électriques unidirectionnelles. Ce brevet fait état d'une logique programmée, bien que non décrite, il ne décrit ni ne suggère aucune logique de contrôle, ni aucun moyen d'assurer la sécurité et l'asservissement.

Un objet de la présente invention est d'obvier aux inconvénients des appareils de l'art antérieur, en fournissant un dispositif électronique à programmation, destiné à stimuler directement les muscles lisses, et notamment ceux des tissus vasculaires, par des courants reproduisant les courants d'action physiologiques.

Un aautre objet de l'invention est de fournir un dispositif électronique à programmation, pour la stimulation des muscles lisses du tissu vasculaire, ce dispositif étant doté d'une part de moyens logiques de contrôle lui permettant de fonctionner de façon autonome ou en liaison avec d'autres instruments, et d'autre part d'assurer une sécurité de fonctionnement.

Encore un objet de la présente invention est de fournir un dispositif électronique à programmation, notamment destiné à la stimulation des muscles lisses, plus particulièrement ceux du tissu vasculaire, le dispositif pouvant être régulé directement à partir d'informations provenant directement du tissu vasculaire à traiter ou de son environnement musculaire, notamment du débit sanguin veineux.

La présente invention a donc pour objet un dispositif électronique à programmation selon la revendication 1, destiné à stimuler la contraction des muscles lisses, notamment des muscles lisses du tissu vasculaire, comportant des moyens permettant de générer des impulsions progressives positives unidirectionnelles, progressives négatives unidirectionnelles ou successivement positives et négatives par train comportant une pluralité d'impulsions, ce dispositif comportant, disposés de façon logique, des moyens de programmation de mise en œuvre comprenant un système de logique programmée et un système de logique de contrôle et de comparaison agencés en boucle logique, des moyens d'intégration définissant la forme du signal et des moyens d'amplification, avantageusement transistorisés et que ces moyens générateurs d'impulsions sont associés à au moins deux électrodes disposées en des endroits appropriés du corps, de façon à permettre aux impulsions de circuler d'au moins une première zone d'application des impulsions à au moins une seconde zone d'application d'impulsions.

Les moyens générateurs sont associés à des électrodes disposées en des endroits appropriés des membres du sujet à traiter de façon à permettre aux impulsions de circuler alternativement d'un membre à l'autre en passant avantageusement par le petit bassin, dans le cas des membres inférieurs.

Le dispositif électronique à programmation selon la présente invention est en outre remarquable par les points suivants: – les moyens de programmation sont constitués par au moins un micro-ordinateur constitué par au moins un microprocesseur associé à au moins une mémoire morte (ROM) et à au moins une mémoire vive (RAM); le micro-processeur est en outre associé à au moins une mémoire programmable (PROM); le micro-processeur est associé à au moins une mémoire effaçable et reprogrammable (RePROM); on a prévu, associés au système de logique de contrôle, des moyens de captage permettant de détecter les paramètres indicateurs des flux de retour et/ou de l'état des tissus musculaires et/ou vasculaires et de leur environnement, à traiter, ces moyens de captage étant associés à des moyens capables par filtrage, décodage, codage, et amplification, de transformer ces paramètres en signaux exploitables par les moyens de programmation; les moyens d'amplification comportent des composants capables de fonctionner en haute fréquence, par exemple des transistors; chaque micro-processeur est associé à un micro-processeur maître.

Des moyens de traitement digital, des dispositifs de coupure de courant, des filtres, empêchent qu'en aucun cas et particulièrement en salle d'opération, des courants parasites puissent influer sur le fonctionnement de l'appareil et préservent la qualité des signaux émis et la sécurité du patient. En outre, les éléments raccordés au patient sont fabriqués en matériaux stérilisables aux gaz et/ou à haute température pour assurer la prophylaxie bactérienne, particulièrement importante dans certaines interventions.

D'autres avantages et caractéristiques de l'invention apparaîtront à la lecture de la description non limitative suivante d'une forme de réalisation de dispositif stimulateur selon l'invention, en référence au dessin annexé, dans lequel:

– la figure 1 est un schéma synoptique d'un dispositif électronique à programmation, destiné à stimuler la contraction des muscles lisses, selon l'invention;

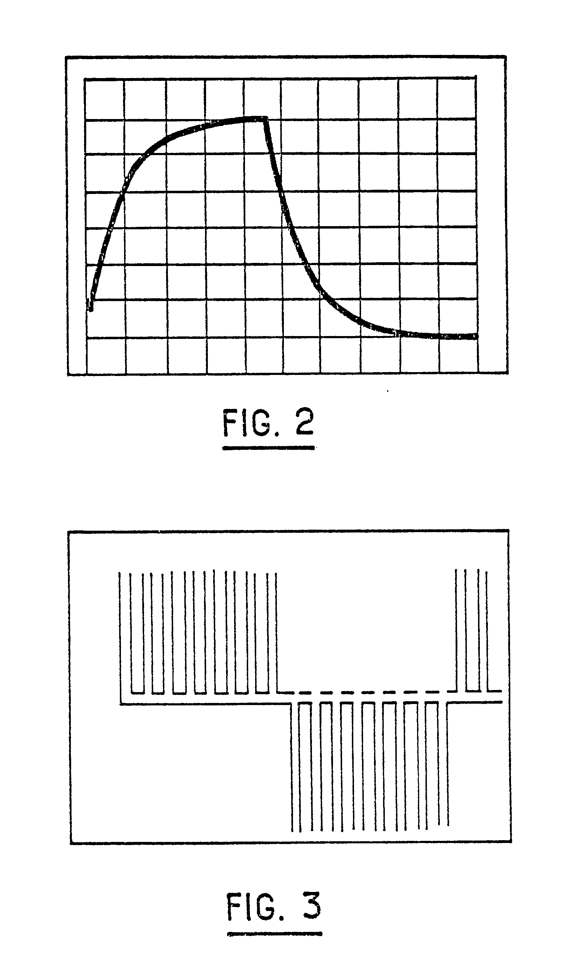

– la figure 2 est une vue d'une impulsion positive progressive obtenue au moyen du dispositif de la figure 1; et

– la figure 3 est une vue d'un train d'impulsions progressives alternées obtenues en mettant en œuvre le dispositif de la figure 1.

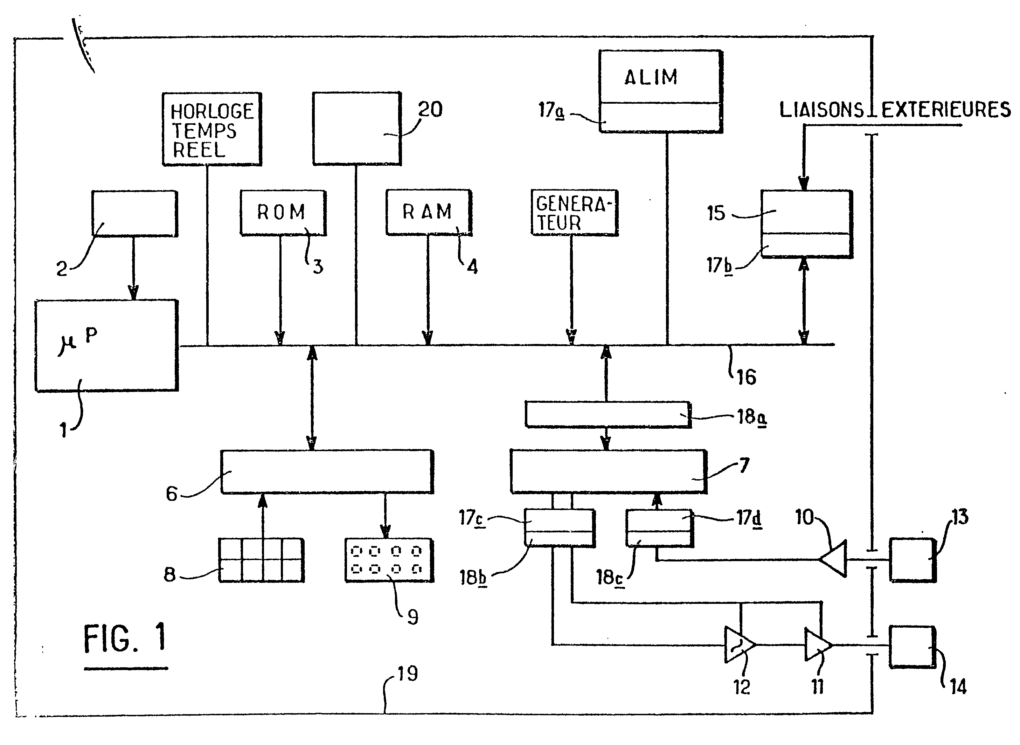

Les moyens de progrmmation du dispositif stimulateur selon la présente invention sont avantageusement constitués, comme représenté sur la figure 1, par un micro-processeur 1 et son circuit d'horloge 2. Ce micro-processeur peut également recevoir les impulsions d'un système d'horloge en temps réel.

Le micro-processeur est associé à une ROM 3 qui contient le programme de séquencement et à une mémoire vive RAM 4.

Une interface 15 assure la liaison sur un connecteur extérieur. Ce connecteur peut être lié à un ordinateur, une banque, un oscillographe, un oscilloscope, un modeur, un graphe, ou tout autre mode de communication aproprié.

L'entrée des commandes s'effectue à l'aide d'un clavier 8 et la lecture des paramètres du système se fait à l'aide d'afficheurs à segments. Le clavier et les afficheurs sont reliés au bus interne 16 par l'intermédiaire de l'interface 6.

Un ensemble de capteurs peuvent être reliés au système par l'interface 7 et après une adaptation (amplificateur 10). Les moyens de captage 13 sont avantageusement constitués par n'inporte quel élément détecteur, par exemple des jauges de contrainte, des jauges thermiques, des jauges rhéologiques, des jauges résistives, des jauges myographiques, des sondes DOPPLER, des compteurs de scintillation dans le cas où l'on voudrait détecter au moyen de traceurs, l'écoulement sanguin dans un tissu donné, etc...

Un ensemble de filtres 17a, 17b, 17c, 17d, fenêtres 18a, 18b, 18c, blindages 19, comparateur 20 protègent l'appareil et les disspositifs d'électrodes 14 et les capteurs 13 contre l'intervention de courants parasites extérieurs (tels ceux générés par un bistouri électrique, un appareil radiologique, etc...) ou de malfonctions internes et externes susceptibles d'altérer le fonctionnement de l'instrument ou la qualité des signaux émis et reçus.

Les électrodes 14, capteurs 13 et leurs câbles sont réalisés en matériaux analergiques résistant aux produits chimiques et températures de stérilisation (comme le P.T.F.E. par exemple).

Le comparateur 20 analyse les signaux émis par les capteurs 13 et doté d'un programme approprié d'échange d'information avec les RAM 4 et ROM 5, déterminé par les résultats de l'expérimentation clinique, renvoie des signaux quantifiés aux électrodes 14. Le comparateur 20 juge le niveau et la qualité des signaux émis vers l'intégrateur 12 et l'ampli 11 ou reçus par les capteurs 13 et ouvre instantanément les circuits en cas de fonctionnement anormal. Les circuits ne peuvent être refermés que lorsque tout est en ordre. Le comparateur est également doté d'un programme spécialisé permettant de tester toutes les fonctions et circuits internes au moyen du clavier 8 des afficheurs 9 et/ou des liaisons externes 15.

Le dispositif générateur de signaux est constitué d'un intégrateur 12 associé à des moyens amplificateurs 11, eux-mêmes reliés à un système d'électrodes 14.

En outre, l'intégrateur 12 et l'amplificateur 11 peuvent être commandés par le micro-processeur 1 ou le comparateur 20; ceci permet de faire varier la forme et l'amplitude des impulsions de sortie.

La diversité de la forme et de l'amplitude des signaux générés permet la stimulation du muscle lisse ou du tissu vasculaire, ainsi que des muscles striés de l'environnement.

De la description qui précède, il est clair que l'utilisation du dispositif de la présente invention peut être mis en œuvre de diverses façons, notamment en ce qui concerne l'utilisation par programmation manuelle et déclenchement au moyen des signaux émis à partir des paramètres détectés au niveau des tissus à traiter, ou bien en programmant le traitement à l'avance au moyen de l'horloge à temps réel associée au micro-processeur 1, non représenté sur les dessins.

Dans les services ou organisations nécessitant l'emploi régulier d'un certain nombre d'appareils, ceux-ci sont économiquement et avantageusement remplacés par un générateur central lié et distribué par un réseau de câbles blindés ou non à des terminaux individuels programmables comprenant la partie nécessaire des fonctions décrites précédemment.

Les signaux délivrés par le dispositif selon l'invention reproduisent les courants physiologiques parcourent les fibres amyéliniques vasomotrices sympathiques qui aboutissent sur les parois et toute l'épaisseur de la veine et de l'endoveine. Ils franchissent les vésicules de jonction et permettent de fermer le circuit électrique. Ces signaux présentent avantageusement les caractéristiques suivantes:

– une constante de temps égale à ou de l'ordre de 40µs;

– une durée d'extinction égale à la durée de l'établissement de l'impulsion;

– une largeur à la base égale à 3 ms – 25 ms + 28 ms;

– une amplitude de crête réglable de 0 à 130 volts et de –130 à 0 v.;

– une période égale à 625 ms – 500 ms + 1500 ms.

Il est préférable que la durée de l'impulsion ne dépasse jamais 80% de la période d'impulsion, tandis que la tension de crête ne dépasse à aucun moment 125% de la valeur de réglage, les variations de la tension de sortie ne produisant aucune modification de la fréquence de réglage. Dans le cas de trains d'impulsions progressives alternées, il est préférable de mettre en œuvre des trains de 8 impulsions (–6, +30) de caractéristiques identiques à celles énoncées précédemment et dont la période de récurrence se situe entre –8 et +20 s, et est par exemple égale à 10 s. Il est également possible, selon l'invention, de mettre en œuvre de très basses, moyennes ou hautes fréquences et/ou d'amplitudes dont les caractéristiques de l'enveloppe sont identiques à celles énoncées précédemment.

Les expérimentations cliniques, auxquelles est soumis le dispositif stimulateur selon l'invention, ont donné des résultats particulièrement intéressants, notamment dans le traitement des maladies thrombovasculaires. Le dispositif stimulateur de l'invention peut être associé à n'importe quel moyen d'exploration fonctionnelle, par exemple un dispositif DOPPLER, un compteur de radiation, un détecteur de radiations, un détecteur théologique et tout dispositif analogue mettant en évidence l'accélération du flux de retour et l'augmentation des indices de remplissage et de vidange du système veineux.

Il est clair que l'invention n'est nullement limitée à la forme de réalisation décrite ci-dessus en référence au dessin annexé, mais qu'elle englobe toutes les modifications et variantes à la portée de l'homme de l'art, issues du même principe de base. C'est ainsi que l'on peut associer au micro-ordinateur décrit ci-dessus, n'importe quels moyens de logique programmée ou de logique de contrôle, par exemple une ou plusieurs mémoires programmables PROM et/ou mémoires effaçables et reprogrammables RePROM, ou bien connecter le micro-processeur sur un micro-ordinateur maître.

Par ailleurs, un micro-processeur maître, non représenté aux dessins, peut être associé, dans le cadre de la présente invention, à un réseau de micro-processeurs programmables individuellement.

Une réalisation simplifiée peut mettre en œuvre de la logique C-MOS.

Selon une autre forme de réalisation simplifiée, excluant les comparateurs, on peut mettre en œuvre une boucle analysant le courant utilisé lors de la stimulation, et coopérant avec un système d'ampèremètre-potentiomètre, affichant les courants faibles utilisés.

Une application thérapeutique de l'invention permet une action fibrinolytique au niveau des cellules sur les prostaglandines intervenant dans les actions antiphlébitiques.

**Revendications**

1. Dispositif électronique à programmation, destiné à stimuler la contraction des muscles lisses vasomoteurs, notamment les fibres musculaires lisses présentes dans le tissu vasculaire comportant des moyens permettant de générer des impulsions progressives positives unidirectionnelles, progressives négatives unidirectionnelles, ou successivement positives et négatives par trains comportant une pluralité d'impulsions alternées, ces moyens générateurs comportant, disposés de façon logique, des moyens de programmation de mise en œuvre comportant un système de logique programmée et un système de logique de contrôle et de comparaison agencés en boucle logique, des moyens d'intégration définissant la forme du signal d'impulsion générée et des moyens d'amplification, et ces moyens générateurs d'impulsions étant associés à deux électrodes disposées en des endroits appropriés du corps, de façon à permettre aux impulsions de circuler d'une première zone d'application des impulsions à une seconde zone d'application d'impulsion, les signaux présentant une forme exponentielle (fig. 2) avec les caractéristiques suivantes:

– une constante de temps égale à ou de l'ordre de 40 microsecondes;

– une durée d'extinction égale à la durée de l'établissement de l'impulsion;

– une largeur à la base d'environ 3 ms;

– une amplitude de crête réglable de 0 à 130 volts et de –130 à 0 volts; et

– une période comprise entre 125 ms et 2125 ms, préférentiellement 625 ms.

2. Dispositif selon la revendication 1, caractérisé par le fait que les moyens de programmation sont constitués par au moins un micro-ordinateur constitué par au moins un micro-processeur associé à au moins une mémoire morte (ROM) et au moins une mémoire vive (RAM).

3. Dispositif selon la revendication 2, caractérisé en ce que le micro-processeur est en outre associé à au moins une mémoire programmable (PROM).

4. Dispositif selon la revendication 2, caractérisé par le fait que le micro-processeur est associé à au moins une mémoire effaçable et reprogrammable (RePROM).

5. Dispositif selon la revendication 1, caractérisé en ce qu'il comporte, associés aux moyens de programmation, des moyens de captage permettant de détecter les paramètres indicateurs de l'état des tissus musculaires et/ou vasculaires, ainsi que de leur environnement, ces moyens de captage étant associés à des moyens capables, par filtrage et amplification, de transformer ces données paramétriques en signaux exploitables par les moyens de programmation.

6. Dispositif selon la revendication 1, caractérisé par le fait que les moyens d'amplification comportent des transistors capables de fonctionner à la fois en haute et basse fréquence, selon besoin.

7. Dispositif selon la revendication 2, caracérisé en ce que chaque micro-processeur est associé à un micro-ordinateur maître.

8. Dispositif selon la revendication 2, caractérisé en ce qu'est prévu un micro-processeur maître, associé à un réseau de micro-processeurs programmables individuellement.

## Claims

1. Programmed electronic device intended to stimulate the contraction of vasomotor involuntary muscles especially the involuntary muscular fibers present in the vascular tissue comprising means enabling to generate unidirectional positive progressive impulses, unidirectional negative progressive impulses, or successively positive and negative impulses by means of sequences comprising a plurality of alternate impulses, said generating means comprising operational programming means consisting of a programmed system arranged as a logical loop, integrating means defining the shape of a generated impulse signal and amplifying means, and said impulse generating means being associated to two electrodes located in convenient locations of the human body as to enable the impulses to circulate from a first application surface area of impulses to a second application surface area of impulses, the whole means being logically arranged, the signals having an exponential shape (Fig. 2) with the following features:
– a time constant equal to or in the range 40 microseconds;
– an extinction duration equal to the duration of impulse generating period of time;
– a width at the base equal to about 3 ms;
– a peak amplitude adjustable from 0 to 130 Volts and from –130 to 0 Volts;
– a period comprised between 125 ms and 2125 ms, preferentially 625 ms.

2. Device according to Claim 1, characterized in that the programming means comprise at least a micro-computer having at least a micro-processor associated with at least a read-only memory (ROM) and at least a random access memory (RAM).

3. Device according to Claim 2, characterized in that the micro-processor is further associated with at least a programmable memory (PROM).

4. Device according to Claim 2, characterized in that the micro-processor is associated with at least an erasable and re-programmable memory (RePROM).

5. Device according to Claim 1, characterized in that it further comprises sensing means associated with the programming means for detecting parameters indicative of the state of the muscular and/or vascular tissues, as well as the environment thereof, said sensing means being associated with means for converting said parametric data to signals able to be used by the programming means, by means of filtering and amplifying sais parameters.

6. Device according to Claim 1, characterized in that the amplifying means comprises transistors able to be used with both higher and lower frequencies, as needed.

7. Device according to Claim 2, characterized in that the micro-processor in associated with a main micro-computer.

8. Device according to Claim 2, characterized in that provision is made for a main micro-processor with is associated with a network of micro-processors which are each programmable.

## Patentansprüche

1. Programmierbare, elektronische Vorrichtung, dazu bestimmt, Muskelkontraktionen glatter Gefäßnerven zu stimulieren; insbesondere glatter Muskelfasern, die im Gefäßgewebe vorhanden sind, wobei Vorrichtung enthält Mittel, die ermöglichen, progressive, positive, einseitige gesteuerte Impulse sowie progressive, negative ebenfalls einseitige gesteuerte Impulse, oder abwechselnd positive und negative Impulse zu erzeugen, mittels Getrieben, die eine Vielheit von aufeinanderfolgenden, sich ablösenden Impulsen enthalten, wobei Impulse erzeugenden Mittel, in einer logischen Schleife angeordnet, erlauben die Anwendung von Programmierungsmitteln, die ein System von programmierter Logik und ein logisches Kontroll- und Vergleichssystem enthalten, sowie Integrationsmittel und Verstärkungsmittel, die die erzeugende Form des Impulssignals definieren, wobei Impuls erzeugenden Mittel, in Verbindung mit zwei, angeeigneten Stellen des Körpers angelegten Eletroden, erlauben den Impulsionen von einer ersten, zu einer zweiten Impulsionsanwendungszone überzugehen und zu zirkulieren, indem die Signale eine exponentielle Form, mit den folgenden Eigenschaften aufweisen (Fig. 2):
einer Zeitkonstante egal 40 ms,
einer Löschungsdauer egal der Ansprechdauer der Impulse,
einer Breite von ungefähr 3 ms,
eines regulierbaren Spitzenausschlags von 0 bis 130 Volt, und von –130 bis 0 Volt,
einer Periode zwischen 125 ms und 2125 ms, vorzugsweise 625 ms.

2. Vorrichtung nach Anspruch 1, dadurch gekennzeichnet, daß die Programmierungsmittel aus mindestens einem Mikrocomputer gebildet sind, der mindestens aus einem Mikroprozessor mit mindestens einem ROM und einem RAM gebildet ist.

3. Vorrichtung nach Anspruch 2, dadurch gekennzeichnet, daß der Mikroprozessor außerdem, mindestens mit einem PROM zusammengestellt ist.

4. Vorrichtung nach Anspruch 2, dadurch gekennzeichnet, daß der Mikroprozessor mindestens mit einem RePROM zusammengestellt ist.

5. Vorrichtung nach Anspruch 1, dadurch gekennzeichnet, daß mit den Programmierungsmitteln, Einfangsmittel kombiniert sind, die ermöglichen Indikationsparameter über den Zustand der Muskel- und Vaskulargewebe und ihrer Umgebung aufzudecken, wobei Einfangsmittel sind, durch Filterung und Verstärkung diese Parameterdaten in Signale umzusetzen, ausnutzbar durch Programmierungsmittel.

6. Vorrichtung nach Anspruch 1, dadurch gekennzeichnet, daß die Verstärkungsmittel einen Transistor enthalten, geeignet zugleich in Hoch- und Niederfrequenz zu funktionieren.

7. Vorrichtung nach Anspruch 2, dadurch gekennzeichnet, daß jeder Mikroprozessor mit einem Hauptcomputer zusammengestellt ist.

8. Vorrichtung nach Anspruch 2, dadurch gekennzeichnet, daß ein Hauptmikroprozessor mit einem individuellen programmierbaren Mikroprozessornetz verbunden ist.

FIG. 1

FIG. 2

FIG. 3